(19) Europäisches Patentamt / European Patent Office / Office européen des brevets

(11) **EP 4 361 601 A1**

(12) **EUROPEAN PATENT APPLICATION**
published in accordance with Art. 153(4) EPC

(43) Date of publication:
**01.05.2024 Bulletin 2024/18**

(21) Application number: **22841729.1**

(22) Date of filing: **24.03.2022**

(51) International Patent Classification (IPC):
**G01N 21/27** (2006.01)   **G01N 21/3554** (2014.01)

(52) Cooperative Patent Classification (CPC):
**G01N 21/359; C21B 7/24; C21C 5/4673;
G01N 21/3554;** C10L 5/02; C10L 2290/18;
C10L 2290/60; G01N 21/55; G01N 2201/0214

(86) International application number:
**PCT/JP2022/014053**

(87) International publication number:
**WO 2023/286379 (19.01.2023 Gazette 2023/03)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **14.07.2021 JP 2021116724**

(71) Applicant: **Kurita Water Industries Ltd.
Tokyo 164-0001 (JP)**

(72) Inventors:
• **HIDAKA, Katsuhiko**
  **Tokyo 164-0001 (JP)**
• **KIKKAWA, Takashi**
  **Tokyo 164-0001 (JP)**
• **NEZAKI, Takasuke**
  **Tokyo 164-0001 (JP)**
• **MASUI, Yukihito**
  **Tokyo 164-0001 (JP)**
• **NORO, Naoki**
  **Tokyo 108-0075 (JP)**
• **TAKARA, Yohei**
  **Tokyo 108-0075 (JP)**

(74) Representative: **TBK
Bavariaring 4-6
80336 München (DE)**

(54) **ESTIMATION SYSTEM, ESTIMATION DEVICE, ESTIMATION METHOD, AND ESTIMATION PROGRAM**

(57)    Provided are an estimation system, an estimation device, an estimation method, and an estimation program that are capable of accurately estimating at least any of the amount of moisture and the content of a chemical agent in a material as a steelmaking raw material and/or a power generation raw material irrespective of the measurement distance and the meteorological condition. According to an aspect of the present invention, an estimation device configured to estimate the amount of moisture in a material as a steelmaking raw material and/or fuel for power generation is provided. The estimation device includes a moisture model information acquisition unit, a moisture index information acquisition unit, and a moisture amount estimation unit. The moisture model information acquisition unit acquires moisture model information indicating the relation between the amount of moisture in the material and a moisture index calculated as a function of the difference between two optical properties of the material for light of two different wavelengths in a wavelength range of 800 nm to 2400 nm. The moisture index information acquisition unit acquires moisture index information indicating the moisture index of the material as an estimation target. The moisture amount estimation unit estimates the moisture amount of the material as an estimation target based on the moisture model information and the moisture index information.

Fig. 1

**Description**

Technical Field

The present invention relates to an estimation system, an estimation device, an estimation method, and an estimation program.

Background Art

The moisture amount of a material such as iron ore or coal used as a steelmaking raw material or fuel for power generation needs to be maintained in an appropriate range. When dried too much, such a material generates powder dust, or when its moisture amount is too much, the material causes clogging at conveyance or needs a large amount of heat for drying, which potentially decreases energy efficiency.

The amount of moisture included in the material needs to be determined to maintain the moisture amount in an appropriate range by spraying water when dried or by spraying a chemical agent such as a water shielding agent when the moisture amount is too large.

For example, PTL1 discloses a method of measuring the moisture amount at the surface of a pile of iron ore by using a near-infrared moisture content meter to determine the amount of moisture included in the material and perform water spraying for preventing powder dust.

Citation List

Patent Literature

PTL1: JP 2008-050076 A

Summary of Invention

Technical Problem

However, in measurement of the amount of moisture in such a material as a steelmaking raw material or power generation raw material, the measured value of the moisture amount is affected by the difference in the measurement distance and the difference in the meteorological condition when the measurement is performed outdoor. Thus, without calibration of the meteorological condition and the measurement distance under the same condition, it is impossible to accurately measure the amount of moisture in a material as a steelmaking raw material or power generation raw material.

To maintain the moisture amount of a steelmaking raw material or a power generation raw material in an appropriate range, it is needed to determine existence of a consolidated layer on a surface layer of the raw material, the consolidated layer being formed of a chemical agent (dust-proof agent) added to the material.

The present invention is made to solve the above-described problem and provides an estimation system, an estimation device, an estimation method, and an estimation program that are capable of accurately estimating at least any of the amount of moisture and the content of a chemical agent in a material as a steelmaking raw material and/or a power generation raw material irrespective of the measurement distance and the meteorological condition.

Solution to Problem

According to an aspect of the present invention, an estimation device configured to estimate the amount of moisture in a material as a steelmaking raw material and/or fuel for power generation is provided. The estimation device includes a moisture model information acquisition unit, a moisture index information acquisition unit, and a moisture amount estimation unit. The moisture model information acquisition unit acquires moisture model information indicating the relation between the amount of moisture in the material and a moisture index calculated as a function of the difference between two optical properties of the material for light of two different wavelengths in a wavelength range of 800 nm to 2400 nm. The moisture index information acquisition unit acquires moisture index information indicating the moisture index of the material as an estimation target. The moisture amount estimation unit estimates the moisture amount of the material as an estimation target based on the moisture model information and the moisture index information.

Specifically, the above-described aspect may be provided in aspects as follows.

In the estimation device, the moisture model information indicates a linear relation between the moisture index and the moisture amount of the material.

In the estimation device, the optical properties are absorptance, reflectance, or transmittance.

In the estimation device, the optical properties of the material as an estimation target are measured by using a hyperspectral camera or a multispectral camera.

In the estimation device, the two optical properties of the material as an estimation target are associated with two-dimensional position information, the moisture index information acquisition unit acquires, in association with the two-dimensional position information, moisture index information indicating the moisture index of the material as an estimation target, and the moisture amount estimation unit estimates, for each two-dimensional position, the moisture amount of the material as an estimation target based on the moisture model information and the moisture index information.

In the estimation device, the optical properties of the material as an estimation target are measured by the hyperspectral camera or the multispectral camera attached to an aerial vehicle.

An estimation device configured to estimate the content of a chemical agent in a material as a steelmaking raw material and/or fuel for power generation, the estimation device including a chemical agent inclusion model information acquisition unit, an inclusion index information acquisition unit, and a content estimation unit, in which: the chemical agent inclusion model information acquisition unit acquires chemical agent inclusion model information indicating the relation between the content or existence of inclusion of a chemical agent in the material and an inclusion index calculated as a function of the difference between two optical properties of the material for light of two different wavelengths in a wavelength range of 800 nm to 2400 nm, the inclusion index information acquisition unit acquires inclusion index information indicating the inclusion index of the material as an estimation target, and the content estimation unit estimates the content or existence of inclusion of a chemical agent in the material as an estimation target based on the chemical agent inclusion model information and the inclusion index information.

An estimation system configured to estimate the amount of moisture in a material as a steelmaking raw material and/or fuel for power generation, the estimation system including a moisture model information acquisition unit, a moisture index information acquisition unit, and a moisture amount estimation unit, in which: the moisture model information acquisition unit acquires moisture model information indicating the relation between the amount of moisture in the material and a moisture index calculated as a function of the difference between two optical properties of the material for light of two different wavelengths in a wavelength range of 800 nm to 2400 nm, the moisture index information acquisition unit acquires moisture index information indicating the moisture index of the material as an estimation target, and the moisture amount estimation unit estimates the moisture amount of the material as an estimation target based on the moisture model information and the moisture index information.

An estimation system configured to estimate the content of a chemical agent in a material as a steelmaking raw material and/or fuel for power generation, the estimation system including a chemical agent inclusion model information acquisition unit, an inclusion index information acquisition unit, and a content estimation unit, in which: the chemical agent inclusion model information acquisition unit acquires chemical agent inclusion model information indicating the relation between the content or existence of inclusion of a chemical agent in the material and an inclusion index calculated as a function of the difference between two optical properties of the material for light of two different wavelengths in a wavelength range of 800 nm to 2400 nm, the inclusion index information acquisition unit acquires inclusion index information indicating the inclusion index of the material as an estimation target, and the moisture amount estimation unit estimates the content or existence of inclusion of a chemical agent in the material as an estimation target based on the chemical agent inclusion model information and the inclusion index information.

An estimation method of estimating the amount of moisture in a material as a steelmaking raw material and/or fuel for power generation, the estimation method including a moisture model information acquiring step, a moisture index information acquiring step, and a moisture amount estimating step, in which: the moisture model information acquiring step acquires moisture model information indicating the relation between the amount of moisture in the material and a moisture index calculated as a function of the difference between two optical properties of the material for light of two different wavelengths in a wavelength range of 800 nm to 2400 nm, the moisture index information acquiring step acquires moisture index information indicating the moisture index of the material as an estimation target, and the moisture amount estimating step estimates the moisture amount of the material as an estimation target based on the moisture model information and the moisture index information.

The estimation method further includes an optical property measuring step and an optical property acquiring step, the optical property measuring step measures the two optical properties of the material as an estimation target by using a hyperspectral camera or a multispectral camera, and the optical property acquiring step acquires the two optical properties of the material as an estimation target.

In the estimation method, the optical properties are associated with two-dimensional position information, the moisture index information acquiring step acquires, in association with two-dimensional position information, moisture index information indicating the moisture index of the material as an estimation target, and the moisture amount estimating step estimates, for each two-dimensional position, the moisture amount of the material as an estimation target based on the moisture model information and the moisture index information.

In the estimation method, the optical property measuring step measures the two optical properties of the material as an estimation target by using the hyperspectral camera or the multispectral camera attached to an aerial vehicle.

An estimation method of estimating the content of a chemical agent in a material as a steelmaking raw material and/or fuel for power generation, the estimation method including a chemical agent inclusion model information acquiring step, an inclusion index information acquiring step, and a content estimating step, in which: the chemical agent inclusion model information acquiring step acquires chemical agent inclusion model information indicating the relation between the content or existence of inclusion of a chemical agent in the material and an inclusion index calculated as a function of the difference between two optical properties of the material for light of two different wavelengths in a wavelength range of 800 nm to 2400 nm, the inclusion index information acquiring step acquires inclusion index information indicating the inclusion index of the material as an estimation target, and the moisture amount estimating step estimates the content or existence of inclusion of a chemical agent in the material as an estimation target based on the chemical agent inclusion model information and the inclusion index information.

An estimation program configured to estimate the amount of moisture in a material as a steelmaking raw material and/or fuel for power generation, the estimation program causing a computer to function as a moisture model information acquisition unit, a moisture index information acquisition unit, and a moisture amount estimation unit, in which: the moisture model information acquisition unit acquires moisture model information indicating the relation between the amount of moisture in the material and a moisture index calculated as a function of the difference between two optical properties of the material for light of two different wavelengths in a wavelength range of 800 nm to 2400 nm, the moisture index information acquisition unit acquires optical property information indicating the moisture index of the material as an estimation target, and the moisture amount estimation unit estimates the moisture amount of the material as an estimation target based on the moisture model information and the moisture index information.

An estimation program configured to estimate the content of a chemical agent in a material as a steelmaking raw material and/or fuel for power generation, the estimation program causing a computer to function as a chemical agent inclusion model information acquisition unit, an inclusion index information acquisition unit, and a content estimation unit, in which: the chemical agent inclusion model information acquisition unit acquires chemical agent inclusion information indicating the relation between the content or existence of inclusion of a chemical agent in the material and an inclusion index calculated as a function of the difference between two optical properties of the material for light of two different wavelengths in a wavelength range of 800 nm to 2400 nm, the inclusion index information acquisition unit acquires inclusion index information indicating the inclusion index of the material as an estimation target, and the moisture amount estimation unit estimates the content or existence of inclusion of a chemical agent in the material as an estimation target based on the chemical agent inclusion model information and the inclusion index information.

However, the present invention is not limited to the above-described aspects.

According to the present invention, it is possible to provide an estimation system, an estimation device, an estimation method, and an estimation program that are capable of accurately estimating at least any of the amount of moisture and the content of a chemical agent in a material as a steelmaking raw material and/or a power generation raw material irrespective of at least one of the measurement distance and the meteorological condition.

Brief Description of Drawings

[Fig. 1] Fig. 1 is a schematic view illustrating an estimation system according to the present embodiment.
[Fig. 2] Fig. 2 is a schematic view illustrating a functional configuration of an estimation device according to the present embodiment.
[Fig. 3] Fig. 3 is a schematic view illustrating a hardware configuration of the estimation device according to the present embodiment.
[Fig. 4] Fig. 4 is a flowchart diagram of an estimation method according to the present embodiment.
[Fig. 5] Fig. 5 is a plot of the actual value of the moisture content against an NDSI value for coal A.
[Fig. 6] Fig. 6 is a plot of the actual value of the moisture content against an NDSI value for coal B.
[Fig. 7] Fig. 7 is a plot of the actual value of the moisture content against an NDSI value for coal C.
[Fig. 8] Fig. 8 is a plot of the actual value of the moisture content against an NDSI value for coal D.
[Fig. 9] Fig. 9 is a plot of the actual value of the moisture content against an NDSI value for iron ore E.
[Fig. 10] Fig. 10 is a plot of the actual value of the moisture content against an NDSI value for iron ore F.
[Fig. 11] Fig. 11 is a plot of the actual value of the moisture content against an NDSI value for iron ore G.

Description of Embodiments

An embodiment of the present invention will be described below with reference to the accompanying drawings. Various characteristic matters described below in the embodiment may be combined with each other.

A computer program for achieving software according to the present embodiment may be provided as a non-transitory computer-readable recording medium, may be provided such that the computer program can downloaded from an external server, or may be provided such that the computer program is activated at an external computer and functions thereof are implemented at a client terminal (what is called cloud computing).

In the present embodiment, "unit" may include, for example, combination of hardware resources implemented by circuits in a broad sense and software information processing specifically achieved by the hardware resources. Various kinds of information handled in the present embodiment is expressed as, for example, a physical value of a signal value indicating voltage or current, the magnitude of a signal value as a binary assembly of bits constituted by 0 or 1, or quantum superposition (what is called quantum bit), and can be communicated or calculated on circuits in a broad sense.

A circuit in a broad sense is a circuit achieved by at least appropriately combining a circuit, a circuitry, a processor, a memory, and the like. Specifically, a circuit in a broad sense includes an application specific integrated circuit (ASIC) and a programmable logic device (for example, a simple programmable logic device (SPLD), a complex programmable logic device (CPLD), and a field programmable gate array (FPGA)).

<Moisture amount estimation>

[Estimation system and estimation device]

An estimation system according to the present embodiment is an estimation system configured to estimate the amount of moisture in a material as a steelmaking raw material and/or fuel for power generation. Specifically, the estimation system includes a moisture model information acquisition unit, a moisture index information acquisition unit, and a moisture amount estimation unit. The moisture model information acquisition unit acquires moisture model information indicating the relation between the amount of moisture in the same material as a material as an estimation target and a moisture index calculated as a function of the difference between two optical properties of the material for light of two different wavelengths in the wavelength range of 800 nm to 2400 nm. The moisture index information acquisition unit acquires moisture index information indicating the moisture index of the material as an estimation target. The moisture amount estimation unit estimates the moisture amount of the material as an estimation target based on the moisture model information and the moisture index information.

The "steelmaking raw material" means a material used as a raw material and fuel for steelmaking at a steelmaking facility such as a steelmaking plant, and examples thereof include coal, iron steel, dust, slag, coke, sinter as well as auxiliary raw materials such as lime stone and dolomite. The "fuel for power generation" means fuel used as fuel for power generation at a power generation facility such as a power plant, and examples thereof include coal and biomass fuel.

The moisture amount is a concept including not only the absolute mass but also the moisture amount of the material per unit mass, in other words, the moisture content.

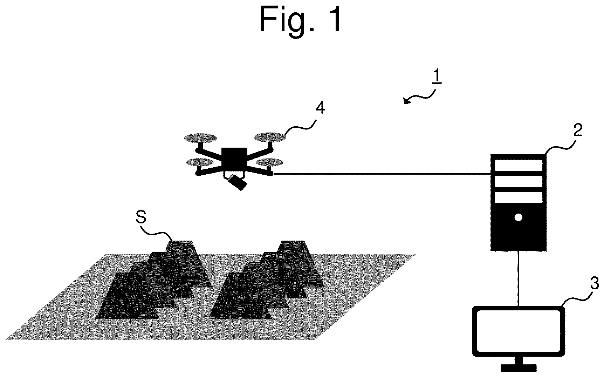

The estimation system according to the present embodiment may include one of or two or more of an optical property information acquisition unit, a moisture index information calculation unit, a moisture model management unit, and an output control units, although the units are not essential. Note that an estimation system including all of these units will be mainly described below with reference to Fig. 1.

[Functional configuration of estimation system]

Fig. 1 is a schematic diagram of the estimation system according to the present embodiment. This estimation system 1 includes an estimation device 2, an output device 3, and an optical property information measurement device 4.

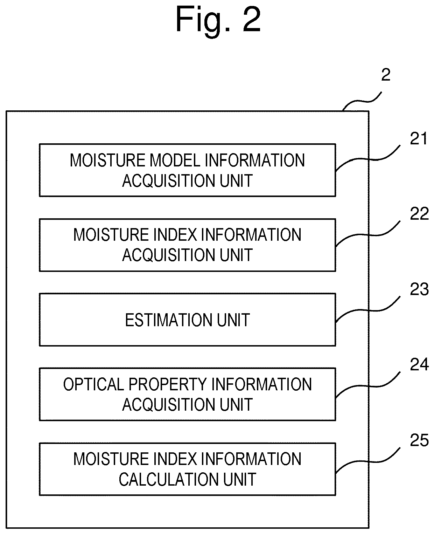

The estimation device 2 controls information processing for estimation of the amount of moisture in a material S in the estimation system 1. Fig. 2 is a schematic diagram illustrating a functional configuration of the estimation device according to the present embodiment. As illustrated in Fig. 2, the estimation device 2 according to the present embodiment includes a moisture model information acquisition unit 21, a moisture index information acquisition unit 22, an estimation unit 23, an optical property information acquisition unit 24, and a moisture index information calculation unit 25. Although not illustrated, the estimation device 2 includes a moisture model management unit and an output control units. Note that the output device 3 is an example of an output unit. The optical property information measurement device 4 is an example of an optical property information measurement unit, which will be described below without distinction.

[Functions of estimation system]

Functions of components of the estimation system 1 will be specifically described below.

(Moisture model information acquisition unit)

The moisture model information acquisition unit 21 acquires moisture model information indicating the relation between the amount of moisture in the material and a moisture index calculated as a function of the difference between two optical properties of the material for light of two different wavelengths in the wavelength range of 800 nm to 2400 nm.

The wavelength range of 800 nm to 2400 nm includes a plurality of wavelength peaks indicating absorption by water molecules. Thus, reflectance measured in the wavelength range is highly correlated with the actual moisture amount of the material S. In particular, the moisture index calculated as a function of the difference between two optical properties for light of two different wavelengths in the wavelength range of 800 nm to 2400 nm reflects influence at the two wavelengths and thus is particularly highly correlated with the actual moisture amount of the material S, and the moisture amount of the material S can be accurately estimated irrespective of the measurement distance and the meteorological condition by using the moisture model information indicating the relation between the moisture index and the moisture amount of the material S.

Note that the above-described wavelength range for selecting two wavelengths is not particularly limited as long as it is in the wavelength range of 800 nm to 2400 nm, but is preferably in the range of 800 nm to 1100 nm, 1160 nm to 1340 nm, or 1440 nm to 2400 nm. The range longer than 1100 nm and shorter than 1160 nm and the range longer than 1340 nm and shorter than 1440 nm potentially suffer contribution of moisture in the atmosphere. Thus, for measurement with reduced influence of the measurement distance and weather (sunny and cloudy weathers), it is preferable to select a wavelength band that is unlikely to be affected by moisture in the atmosphere. Specifically, light in the wavelength ranges of 1110 to 1150 nm and 1350 to 1430 nm is likely to be affected by the atmospheric moisture, and thus it is preferable to exclude these wavelength ranges. By using the optical properties of the material S for light in the wavelength ranges of 800 nm to 1100 nm, 1160 nm to 1340 nm, and 1440 nm to 2400 nm, it is possible to more accurately estimate the moisture amount of the material S irrespective of the measurement distance and the meteorological condition.

The optical properties are not particularly limited but are preferably absorptance, reflectance, or transmittance, and more preferably reflectance.

The "function of the difference between two optical properties of the material for light of two different wavelengths in the wavelength range of 800 nm to 2400 nm" (hereinafter also referred to as a "difference function") will be described below. A case in which 1050 nm and 1330 nm are selected as the two light wavelengths and reflectance is selected as the optical properties will be specifically described as an example. The difference between the two optical properties is expressed as "$R_{1330} - R_{1050}$", where $R_{1050}$ represents the reflectance for light of the wavelength of 1050 nm and $R_{1330}$ represents the reflectance for light of the wavelength of 1330 nm. The difference function is expressed as $f(R_{1330} - R_{1050})$.

When the above-described function is generalized, the difference between the two optical properties is expressed as "$O_j - O_i$" and the difference function is expressed as $f(O_j - O_i)$, where $O_i$ and $O_j$ represent the optical properties for light of wavelengths i nm and j nm, respectively. Note that $O_i$ and $O_j$ may be identical optical properties or different optical properties. The "identical optical properties" means, for example, a case in which $O_i$ and $O_j$ are both reflectance, and the "different optical properties" means, for example, a case in which $O_i$ is reflectance and $O_j$ is absorptance.

The difference function is not limited to a particular form as long as it is a function of "$O_j - O_i$", and may be a function such as $O_j - O_i$, $C(O_j - O_i)$, $C/(O_j - O_i)$, $C^{O_j - O_i}$, $e^{O_j - O_i}$, or $\log(O_j - O_i)$ (in the expressions, C is an optional constant). Moreover, the difference function may additionally include $O_i$ and $O_j$ in its expression and may be, for example, Expressions (1) to (3) below.

$$\frac{O_j - O_i}{O_j} \qquad \cdots (1)$$

$$\frac{O_j - O_i}{O_i} \qquad \cdots (2)$$

$$\frac{O_j - O_i}{O_j + O_i} \qquad \cdots (3)$$

In an embodiment, a normalization difference spectral index (NDSI) may be used as the difference function. Specifically, when $R_i$ and $R_j$ represent reflectance for light of wavelengths i nm and j nm, respectively, the normalization difference spectral index is expressed as Expression (4) below.

$$NDSI = \frac{R_j - R_i}{R_j + R_i} \qquad \cdot \cdot \cdot (4)$$

The moisture model information is not particularly limited as long as it indicates the relation between the moisture index and the moisture amount of the material, but may be, for example, a function or look-up table indicating the relation between the moisture index and the moisture amount, or a model having completed learning of the relation.

The following describes an exemplary method of producing a function indicating the relation between the moisture index and the moisture amount of the material in a case in which the function is used as the moisture model information. A plurality of materials with known moisture amounts that are different from one another to cover an expected range of the moisture amount (at least one moisture amount larger than an expected upper limit of the range and at least one moisture amount smaller than an expected lower limit thereof are preferably included, but the present invention is not limited thereto) are prepared. For each material, the wavelength range of 800 nm to 2400 nm is divided into wavelengths at a constant interval (for example, 10 nm), each two of the divided wavelengths are selected, and the optical properties of the material with a known moisture amount for the wavelengths are measured. The difference between two optical properties for each two wavelengths and the moisture amount (known) are subjected to regression analysis to determine two wavelengths with which the coefficient of determination is particularly high, and the function for the wavelengths is used as the function indicating the relation between the moisture index and the moisture amount of the material. Moreover, the measurement distance and the meteorological condition may be changed for the function to increase the accuracy of estimation by using combination of two wavelengths with which the coefficient of determination is equal to or larger than a certain value (for example, $R^2 > 0.8$). Furthermore, in a case in which the moisture amount of the material is to be estimated irrespective of the kind of the material, the kind of the material may be changed to determine combination of two wavelengths with which the coefficient of determination is equal to or larger than a certain value (for example, $R^2 > 0.8$).

As described above, the wavelength range for selecting two wavelengths is not particularly limited as long as it is in the wavelength range of 800 nm to 2400 nm, but is preferably equal to or longer than 850 nm, or equal to or longer than 900 nm. The wavelength range for selecting two wavelengths is preferably equal to or shorter than 2300 nm, equal to or shorter than 2200 nm, equal to or shorter than 2100 nm, equal to or shorter than 2000 nm, equal to or shorter than 1900 nm, equal to or shorter than 1800 nm, or equal to or shorter than 1700 nm.

The interval of division is not particularly limited but is preferably equal to or longer than 5 nm, equal to or longer than 6 nm, equal to or longer than 7 nm, equal to or longer than 8 nm, equal to or longer than 9 nm, or equal to or longer than 10 nm. A moisture model can be easily produced when the interval of division is equal to or longer than a necessary amount. Moreover, the interval of division is preferably equal to or shorter than 50 nm, equal to or shorter than 45 nm, equal to or shorter than 40 nm, equal to or shorter than 30 nm, equal to or shorter than 20 nm, equal to or shorter than 15 nm, or equal to or shorter than 10 nm. A moisture model can be accurately produced when the interval of division is equal to or shorter than a necessary amount.

The regression analysis is not particularly limited but may be single regression analysis, multiple regression analysis, or the like.

In an embodiment, the moisture model information is preferably a model of a linear relation between the moisture index and the moisture amount of the material. In a case in which the moisture model information of the linear relation is produced, a moisture model may be produced by, for example, single regression analysis.

[Moisture index information acquisition unit]

The moisture index information acquisition unit 22 acquires moisture index information indicating the moisture index of the material S as an estimation target.

The moisture index is calculated by, for example, the moisture index information calculation unit 23 to be described later.

A material having the moisture index acquired by the moisture index information acquisition unit 22 is the material S as an estimation target of the moisture amount. A material used to produce the moisture model information is a material as a specimen having a moisture amount that is known in advance.

The moisture index information acquired by the moisture index information acquisition unit 22 is calculated from optical properties measured at the same wavelengths as two wavelengths related to two optical properties used to produce the moisture model information.

[Moisture amount estimation unit]

The moisture amount estimation unit 23 estimates the moisture amount of the material S as an estimation target based on the moisture model information and the moisture index information.

In a case in which, for example, the function indicating the relation between the moisture index and the moisture amount of the material is used as the moisture model information as described above, the moisture amount estimation unit 23 can estimate the moisture index by substituting the moisture index information acquired by the moisture index information acquisition unit 22 into the function.

[Optical property information acquisition unit]

The optical property information acquisition unit 24 acquires optical property information indicating two optical properties of the material S as an estimation target.

The optical property information is measured by, for example, the optical property information measurement device 4 to be described later.

A material having the optical properties acquired by the optical property information acquisition unit 24 is the material S as an estimation target of the moisture amount. A material used to produce the moisture model information is a material as a specimen having a moisture amount that is known in advance. Note that the measurement distance and the meteorological condition of measurement may be the same or different between the materials. The estimation device according to the present embodiment can accurately estimate the content of the material even when the measurement distance and the meteorological condition of measurement are not the same.

The optical property information acquired by the optical property information acquisition unit 24 is optical properties measured at the same wavelengths as two wavelengths related to two optical properties used to produce the moisture model information.

The optical property information acquired by the optical property information acquisition unit 24 and two optical properties used to calculate the difference function are preferably identical optical properties but may be different optical properties when can be mutually converted.

[Moisture index information calculation unit]

The moisture index information calculation unit 25 calculates the moisture index of the material S as an estimation target based on the two optical properties of the material S as an estimation target for two wavelengths and the difference function.

Specifically, the moisture index of the material S as an estimation target is calculated by, for example, substituting the two optical properties of the material S as an estimation target for two wavelengths into the difference function that is set in advance.

The two optical properties used by the moisture index information calculation unit 25 may be the optical properties acquired by the optical property information acquisition unit 24.

[Moisture model information management unit]

A moisture model information management unit stores and manages the moisture model information.

The moisture model information management unit may be various storage devices.

[Output control units]

The output control units controls output of the moisture amount estimated for the material S by the estimation unit 23.

[Output unit]

The output unit 3 outputs the moisture amount estimated for the material S by the estimation unit 23.

Specifically, the method of outputting by the output unit 3 depends on the method of acquisition by the optical property information measurement device 4 to be described later: in a case in which the material S is disposed in a wide range such as a pile of the material S, the optical properties may be measured for each of constant ranges (areas) into which the range is divided and the estimated moisture amount may be output for each specific range (area); or in a case in which the material S is disposed in a wide range such as a pile of the material S, the optical properties may be measured for each of constant ranges (areas) into which the range is divided and all or some of the optical properties may be summed to output the optical properties in a larger range. In a case in which the optical property measurement range is divided into constant ranges (areas), the measurement range and the output range may be equally or unequally divided and may be divided into constant units such as piles to measure the optical properties and output the estimated moisture amount, and for example, the measurement range may be equally divided into units smaller than a pile and the divided ranges in a range including the pile may be summed to output the estimated moisture amount for the pile. As described above, the optical property measurement range and the manner of its division, and the output range and the manner of its division may be combined as appropriate in accordance with usage or the like.

The division may be performed according to the magnitude of the moisture amount, and a pile may be colored in an image or schematic diagram of a yard or may be indicated with characters.

[Optical property information measurement unit]

The optical property information measurement unit 4 measures the optical property information of the material S as an estimation target.

Since the optical property measurement range and the manner of its division may be selected as appropriate in accordance with usage or the like as described above, the optical property information measurement unit 4 may be selected as appropriate in accordance with the optical property measurement range and the manner of its division and is not particularly limited as long as the unit can measure optical properties. For example, in a case in which a pile of the material S in a yard is measured, a hyperspectral camera or a multispectral camera may be used as the optical property information measurement unit. In this case, the hyperspectral camera or the multispectral camera needs perform measurement from the overhead space of the yard. Thus, such an optical property information measurement unit may be attached and used on an aerial vehicle (for example, an unmanned aerial vehicle UAV such as a drone)) capable of flying at a position higher than the maximum height of the pile of the material S, or on a boom such as a reclaimer that can be extended up to a position higher than the maximum height of the pile. Accordingly, in such a case, the optical properties of the material S as an estimation target are measured by using a hyperspectral camera or a multispectral camera, and particularly, measured by using a hyperspectral camera or multispectral camera attached on an aerial vehicle.

In a case in which the optical property information measurement unit is attached and used on an aerial vehicle or a boom, for example, the two optical properties of the material as an estimation target are preferably associated with two-dimensional position information. The "two-dimensional position information" means position information on a two-dimensional plane in a plan view of a ground such as a yard from its overhead space. When the optical properties are "associated with the two-dimensional position information", it is meant that the optical properties exist in a state in which a two-dimensional position to which the optical properties belong can be specified. In such a case, the moisture index information acquisition unit acquires the moisture index information indicating the moisture index of the material as an estimation target in association with the two-dimensional position information, and the moisture amount estimation unit estimates the moisture amount of the material as an estimation target for each two-dimensional position based on the moisture model information and the moisture index information.

The height at which the optical property information measurement unit 4 is disposed is not particularly limited but the optical property information measurement unit 4 is preferably disposed at a position higher than a yard by 20 m to 200 m inclusive. Specifically, the height at which the pile information measurement unit 4 is disposed may be, for example, 25 m or higher, 30 m or higher, 35 m or higher, 40 m or higher, 45 m or higher, 50 m or higher, 55 m or higher, 60 m or higher, 65 m or higher, 70 m or higher, 75 m or higher, 80 m or higher, 85 m or higher, 90 m or higher, 95 m or higher, 100 m or higher, 105 m or higher, 110 m or higher, 115 m or higher, 120 m or higher, 125 m or higher, 130 m or higher, or 135 m or higher or may be 195 m or lower, 190 m or lower, 185 m or lower, 180 m or lower, 175 m or lower, 170 m or lower, 165 m or lower, or 160 m or lower.

The optical property information measured by the optical property information measurement unit 4 may be transmitted through communication with the optical property information acquisition unit 24, or a recording medium may be attached on the optical property information measurement unit 4 to record pile information therein, and the pile information may be acquired by the optical property information acquisition unit 24 through the recording medium.

With such an estimation system, at least any of the amount of moisture and the content of a chemical agent in the material S as a steelmaking raw material or a power generation raw material can be accurately estimated irrespective of the measurement distance and the meteorological condition. Then, various kinds of responses can be performed based on this estimation result.

As a specific response, for example, addition of a dust-proof agent that reduces powder dust generation or water spraying is performed when the moisture amount of the material S is small and powder dust is likely to be generated, and addition of the dust-proof agent and water spraying are not performed when the moisture amount of the material S is large.

The dust-proof agent is not particularly limited but may be wax emulsion solution, resin emulsion solution, silicone oil, mineral oil, heavy oil, or the like. Wax emulsion is categorized to natural wax and synthesis wax as the raw material of wax. The natural wax is not particularly limited but may be, for example, animal-based wax (such as beeswax or spermaceti), plant-based wax (such as carnauba wax, rice wax, or candelilla wax), petroleum-based wax (such as paraffin wax or micro crystallin wax), or mineral-based wax (such as montan wax or ceresin wax). The synthesis wax is not particularly limited but may be, for example, polyethylene wax, modified natural wax, or hardened oil such as grease. Emulsion resin of the resin emulsion solution is not particularly limited but may be, for example, acrylic resin, acrylic copolymer resin, vinyl acetate-type resin, synthetic rubber, urethane resin, asphalt (emulsifier), acrylic styrene emulsion, styrene butadiene emulsion, ethylene acetic acid vinyl emulsion, or versatate acrylic acid. The silicone oil may be, for example, dimethyl silicone oil or organic functionalized silicone oil (with a functional group such as an amino group, an epoxy group, a mercapto group, a phenyl group, a long-chain alkyl group, or a hydrogen group). Note that the dust-proof agent made of such resin emulsion solution forms a hydrophobic film on the surface of a pile.

Addition of a water shielding agent may be performed by using, for example, a water shielding agent adding device or a water shielding agent addition unit (both not illustrated).

Water is sprayed when the moisture amount of the material S is large.

The water spraying may be performed by using, for example, a water spraying device or a water spraying unit (both not illustrated).

[Hardware configuration of estimation device]

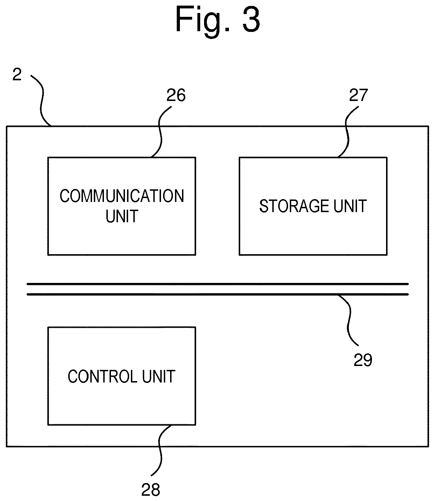

Fig. 3 is a schematic view illustrating a hardware configuration of the estimation device 2 according to the present embodiment. As illustrated in Fig. 3, the estimation device 2 includes a communication unit 26, a storage unit 27, and a control unit 28, and these constituent elements are electrically connected to one another through a communication bus 29 inside the estimation device 2. The constituent elements will be described below.

The communication unit 26 preferably has a wired communication means of USB, IEEE 1394, Thunderbolt, wired LAN network communication, or the like but may include wireless LAN network communication, mobile communication such as 3G, LTE, or 5G, Bluetooth (registered trademark) communication, or the like as necessary. The communication unit 26 more preferably performs a set of these communication means. With this configuration, communication of information and commands is executed with another instrument capable of communicating with the estimation device 2.

The storage unit 27 stores various kinds of information defined by the above description. The storage unit 27 may be implemented as, for example, a storage device such as a solid state drive (SSD), or a memory such as a random access memory (RAM) configured to store temporarily necessary information (such as arguments and arrays) related to calculation of computer programs. Alternatively, the storage unit 27 may be combination thereof. The storage unit 27 also stores various computer programs that are readable by the control unit 28 to be described later.

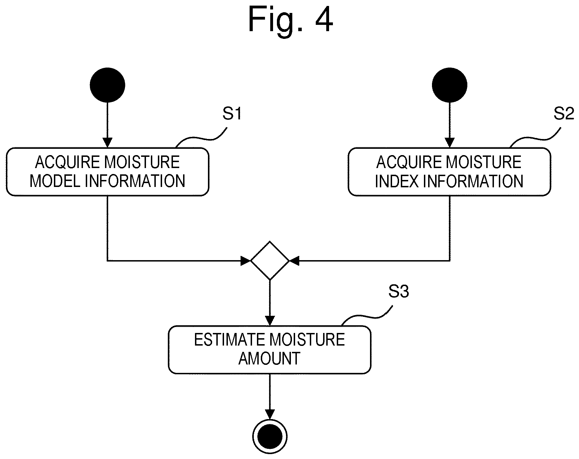

The control unit 28 performs processing and control of the entire operation of the estimation device 2. The control unit 28 is, for example, a central processing unit (CPU; not illustrated). The control unit 28 implements various kinds of functions of the estimation device 2 by reading predetermined computer programs stored in the storage unit 27. In other words, information processing by software (stored in the storage unit 27) is specifically implemented by hardware (the control unit 28) and executed as functional components of the control unit 28 as illustrated in Fig. 4. Note that although Fig. 4 illustrates the single control unit 28, the present invention is not limited thereto in reality and a plurality of control units 28 may be provided for respective functions or a single control unit may be combined with a plurality of control units.

[Estimation method]

An estimation method according to the present embodiment is an estimation method of estimating the amount of moisture in a material as a steelmaking raw material and/or fuel for power generation. Specifically, the estimation method includes a moisture model information acquiring step, a moisture index information acquiring step, and a moisture amount estimating step. The moisture model information acquiring step acquires moisture model information indicating the relation between the amount of moisture in the material and a moisture index calculated as a function of the difference between two optical properties of the material for light of two different wavelengths in the wavelength range of 800 nm to 2400 nm, the moisture index information acquiring step acquires moisture index information indicating the moisture index of the material as an estimation target, and the moisture amount estimating step estimates the moisture amount of the material as an estimation target based on the moisture model information and the moisture index information.

Fig. 4 is a flowchart diagram of the estimation method according to the present embodiment. As illustrated in Fig. 4, in the estimation method according to the present embodiment, the moisture model information is acquired (moisture model information acquiring step S1), the moisture index information is acquired (moisture index information acquiring step S2), the amount of moisture in the material is estimated based on the moisture model information and the moisture index information as input information (moisture amount estimating step S3). The moisture model information acquiring step S1 and the moisture index information acquiring step S2 may be executed in any order: the moisture model information acquiring step S1 may be executed first, the moisture index information acquiring step S2 may be executed first, or the moisture model information acquiring step S1 and the moisture index information acquiring step S2 may be simultaneously executed.

The moisture model information acquiring step, the moisture index information acquiring step, and the moisture amount estimating step are the same as operations of the moisture model information acquisition unit, the moisture index information acquisition unit, and the moisture amount estimation unit described above, and thus specific description thereof is omitted.

Note that the estimation method according to the present embodiment may include an optical property information acquiring step, a moisture index information calculating step, a moisture model managing step, an output controlling step, an outputting step, an optical property information measuring step, a water shielding agent adding step, and a water spraying step. These steps are the same as respective operations of the optical property information acquisition unit, the moisture index information calculation unit, the moisture model management unit, the output control units, the output unit, the optical property information measurement unit, the water shielding agent addition unit, and the water spraying unit, and thus specific description is omitted.

[Estimation program]

An estimation program according to the present embodiment is an estimation program configured to estimate the amount of moisture in a material as a steelmaking raw material and/or fuel for power generation. Specifically, the estimation program causes a computer to function as the moisture model information acquisition unit, the moisture index information acquisition unit, and the moisture amount estimation unit. The moisture model information acquisition unit acquires moisture model information indicating the relation between the amount of moisture in the material and a moisture index calculated as a function of the difference between two optical properties of the material for light of two different wavelengths in the wavelength range of 800 nm to 2400 nm. The moisture index information acquisition unit acquires optical property information indicating the moisture index of the material as an estimation target. The moisture amount estimation unit estimates the moisture amount of the material as an estimation target based on the moisture model information and the moisture index information.

<Estimation of content of chemical agent or existence of inclusion>

The content of a water shielding agent as an organic treatment agent in the material can be accurately estimated by the same method as the above-described estimation of the amount of moisture in the material.

Note that the above description related to estimation of the amount of moisture in the material (including that described as a non-essential element) can be all applied to estimation of the content of a chemical agent in the present section. In this case, "moisture amount" related to estimation of the amount of moisture in the material is applied as "content of the chemical agent", "moisture model information" is applied as "inclusion model information", and "moisture index" is applied as "inclusion index". Estimation of existence of inclusion will be described later in detail.

[Estimation system]

The estimation system according to the present embodiment is an estimation system configured to estimate the content of a chemical agent in a material as a steelmaking raw material and/or fuel for power generation. Specifically, the estimation system includes a chemical agent inclusion model information acquisition unit, an inclusion index information acquisition unit, and a content estimation unit. The chemical agent inclusion model information acquisition unit acquires chemical agent inclusion model information indicating the relation between the content or existence of inclusion of the chemical agent in the material and an inclusion index calculated as a function of the difference between two optical properties of the material for light of two different wavelengths in the wavelength range of 800 nm to 2400 nm. The inclusion index information acquisition unit acquires inclusion index information indicating the inclusion index of the material as an estimation target. The moisture amount estimation unit estimates the content or existence of inclusion of the chemical agent in the material as an estimation target based on the chemical agent inclusion model information and the inclusion index information.

[Estimation device]

The estimation device according to the present embodiment is an estimation device configured to estimate the content of a chemical agent in a material as a steelmaking raw material and/or fuel for power generation. Specifically, the estimation device includes a chemical agent inclusion model information acquisition unit, an inclusion index information acquisition unit, and a content estimation unit. The chemical agent inclusion model information acquisition unit acquires chemical agent inclusion model information indicating the relation between the content or existence of inclusion of the chemical agent in the material and an inclusion index calculated as a function of the difference between two optical properties of the material for light of two different wavelengths in the wavelength range of 800 nm to 2400 nm. The inclusion index information acquisition unit acquires inclusion index information indicating the inclusion index of the material as an estimation target. The content estimation unit estimates the content or existence of inclusion of the chemical agent in the material as an estimation target based on the chemical agent inclusion model information and the inclusion index information.

[Estimation method]

The estimation method according to the present embodiment is an estimation method of estimating the content of a chemical agent in a material as a steelmaking raw material and/or fuel for power generation. Specifically, the estimation method includes a chemical agent inclusion model information acquiring step, an inclusion index information acquiring step, and a content estimating step. The chemical agent inclusion model information acquiring step acquires chemical agent inclusion model information indicating the relation between the content or existence of inclusion of the chemical agent in the material and an inclusion index calculated as a function of the difference between two optical properties of the material for light of two different wavelengths in the wavelength range of 800 nm to 2400 nm. The inclusion index information acquiring step acquires inclusion index information indicating the inclusion index of the material as an estimation target. The moisture amount estimating step estimates the content or existence of inclusion of the chemical agent in the material as an estimation target based on the chemical agent inclusion model information and the inclusion index information.

[Estimation program]

The estimation program according to the present embodiment is an estimation program configured to estimate the content of a chemical agent in a material as a steelmaking raw material and/or fuel for power generation. Specifically, the estimation program causes a computer to function as a chemical agent inclusion model information acquisition unit, an inclusion index information acquisition unit, and a content estimation unit. The chemical agent inclusion model information acquisition unit acquires chemical agent inclusion information indicating the relation between the content or existence of inclusion of the chemical agent in the material and an inclusion index calculated as a function of the difference between two optical properties of the material for light of two different wavelengths in the wavelength range of 800 nm to 2400 nm. The inclusion index information acquisition unit acquires inclusion index information indicating the inclusion index of the material as an estimation target. The moisture amount estimation unit estimates the content or existence of inclusion of the chemical agent in the material as an estimation target based on the chemical agent inclusion model information and the inclusion index information.

In the estimation of existence of inclusion of a chemical agent, for example, a threshold value for the inclusion index may be used as the chemical agent inclusion model information. In a case in which the threshold value is used as the chemical agent inclusion model information, the chemical agent inclusion model information can be calculated by comparing the inclusion index of a material containing the chemical agent (with a known content) with the inclusion index of a material not containing the chemical agent. In this case, the moisture amount estimation unit can estimate existence of inclusion of the chemical agent depending on whether the inclusion index of the material as an estimation target is equal to or larger than the threshold value (or is larger than the threshold value).

The present invention is not limited by the above-described embodiments in any way but may be modified.

Examples

The present invention will be more specifically described below with reference to examples, but the present invention is not limited by the examples below in any way.

Example 1

In a preliminary experiment, the moisture amount was adjusted to 3, 5, 7, and 9 mass% for coal, and the reflection spectrum thereof in the wavelength range of 900 to 1700 nm was measured by using a multispectral camera. Reflectance for 81 wavelengths at the interval of 10 nm (900, 910, 920, ... 1680, 1690, 1700 nm) in the wavelength range was substituted into $R_i$ and $R_j$ in Expression (4), an NDSI value was calculated for each of 6561 combinations of 81 for $R_i$ and 81 for $R_j$, and single regression analysis was performed by using the moisture amounts of the above-described four specimens. From results of the experiment, i = 1020 nm and j = 1330 nm were selected as a combination of wavelengths with which the coefficient of determination is high.

A certain amount of a specimen with an adjusted moisture content for each of coals A to D of mutually different kinds was measured and placed on a flat plate, and the reflection spectrum thereof in the wavelength range of 900 to 1700 nm was measured in each of sunny and cloudy weathers by using a multispectral camera (SIS-I manufactured by EBA JAPAN CO., LTD.). In the reflection spectrum, i = 1020 nm and j = 1330 nm were selected as two wavelengths, and the relation between the NDSI value calculated by using Expression (4) and the actual value of the moisture content of the coal was investigated.

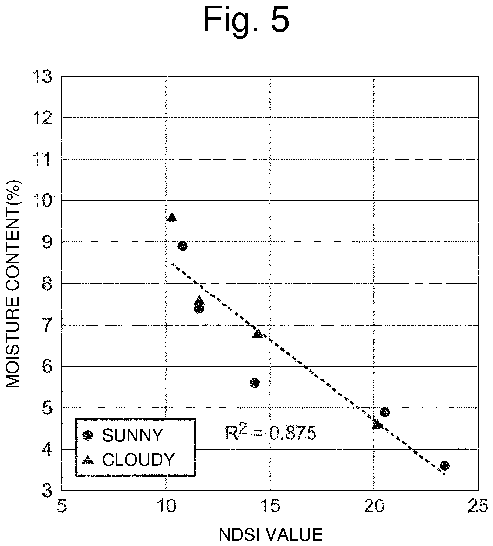

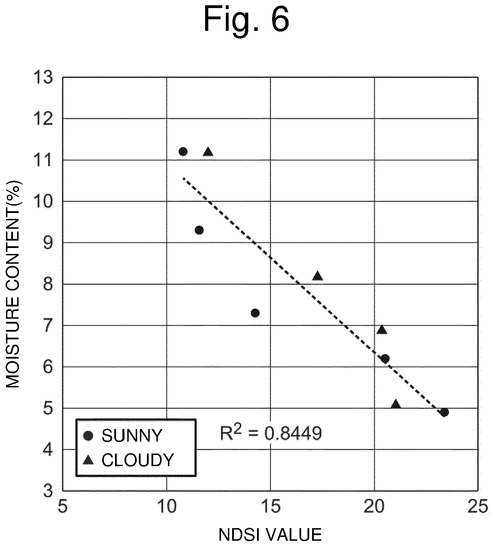

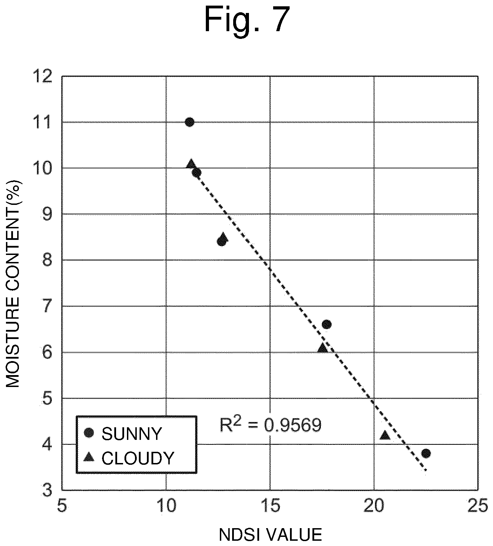

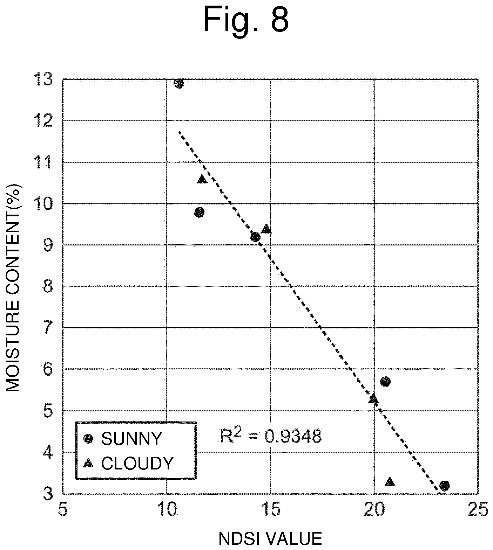

Fig. 5 is a plot of the actual value of the moisture content against the NDSI value for coal A. Fig. 6 is a plot of the actual value of the moisture content against the NDSI value for coal B. Fig. 7 is a plot of the actual value of the moisture content against the NDSI value for coal C. Fig. 8 is a plot of the actual value of the moisture content against the NDSI value for coal D.

For coals A to D, the coefficient $R^2$ of determination was 0.875 (coal A), 0.8449 (coal B), 0.9569 (coal C), and 0.9348 (coal D), and thus it was found that the actual value of the moisture content and the NDSI value for the coals are highly correlated irrespective of sunny and cloudy weathers.


Example 2

A preliminary experiment was performed by using iron ore in place of coal, and i = 950 nm and j = 1330 nm were selected as two wavelengths.

A certain amount of a specimen with an adjusted moisture content for each of iron ores E to G of mutually different kinds was measured and placed on a flat plate, and the reflection spectrum thereof in the wavelength range of 900 to 1700 nm was measured in each of sunny and cloudy weathers by using a multispectral camera. In the reflection spectrum, i = 1020 nm and j = 1330 nm were selected as two wavelengths, and the relation between the NDSI value calculated by using Expression (4) and the actual value of the moisture content of the coal was investigated.

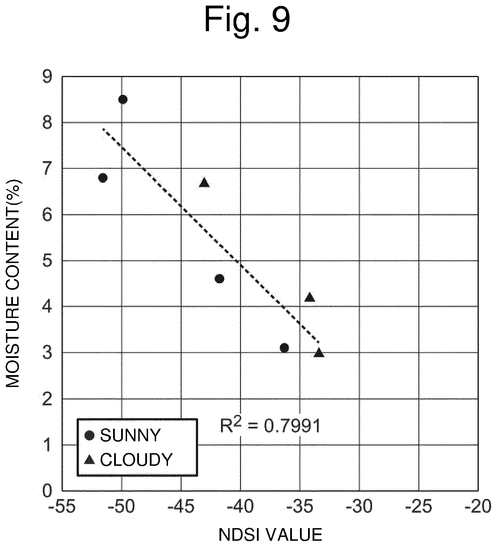

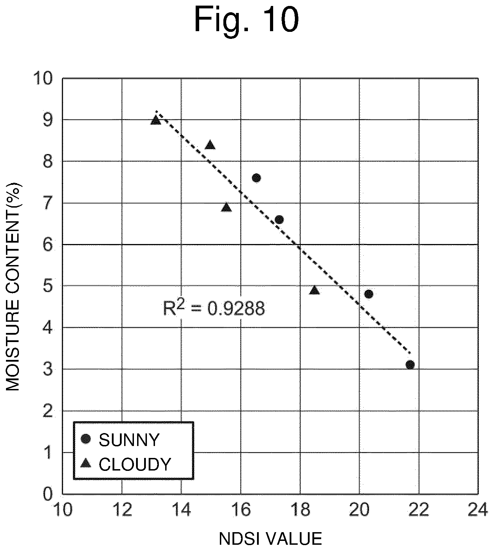

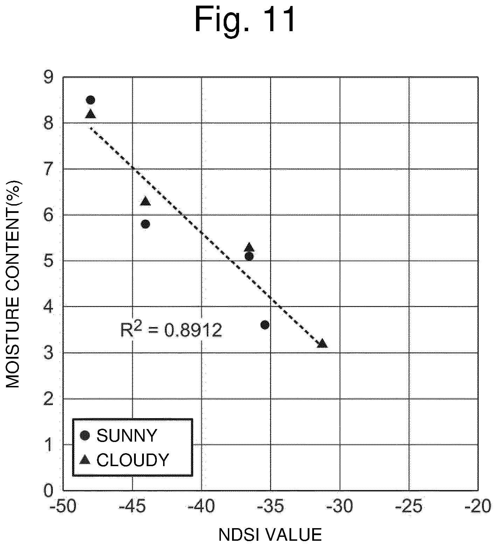

Fig. 9 is a plot of the actual value of the moisture content against the NDSI value for iron ore E. Fig. 10 is a plot of the actual value of the moisture content against the NDSI value for iron ore F. Fig. 11 is a plot of the actual value of the moisture content against the NDSI value for iron ore G.

For iron ores E to G, the coefficient $R^2$ of determination was 0.7991 (iron ore E), 0.9288 (iron ore F), and 0.8912 (iron ore G), and thus it was found that the actual value of the moisture content and the NDSI value for the iron ores are highly correlated irrespective of sunny and cloudy weathers.


Example 3

A chemical agent (mixed emulsion aqueous solution of acetic-acid-vinyl acrylic-acid-ester copolymer and acrylic styrene copolymer as a dust-proof agent; 10 mass%) was sprayed onto coals H to K on a flat plate by an vaporizer and adjusted to 2 L/$m^3$-coal, and then dried at room temperature for two days to produce coals H to K containing the chemical agent.

For one specimen of each of coals H to K containing no chemical agent and two specimens of each of coals H to K containing the chemical agent, the NDSI value was calculated with a combination of wavelengths i = 1020 nm and j = 1330 nm and a combination of wavelengths i = 950 nm and j = 1330 nm as in Example 1. Table 1 lists results of the calculation.

From Table 1, it was found that inclusion of the chemical agent in coals H to K can be estimated by setting the threshold value of the NDSI value to, for example, 16.

[Table 1]

|  | Inclusion of chemical agent | NDSI value (1020 nm/1330 nm) | NDSI value (950 nm/1330 nm) |
|---|---|---|---|
| Coal H | Yes | 9.4 | 7.4 |
| Coal H | Yes | 8.0 | 5.9 |
| Coal H | No | 22.7 | 24.8 |
| Coal I | Yes | 8.9 | 7.0 |
| Coal I | Yes | 6.6 | 4.3 |
| Coal I | No | 21.7 | 23.0 |
| Coal J | Yes | 5.3 | 2.3 |
| Coal J | Yes | 4.8 | 1.7 |
| Coal J | No | 21.1 | 23.8 |
| Coal K | Yes | 9.5 | 7.8 |

(continued)

|  | Inclusion of chemical agent | NDSI value (1020 nm/1330 nm) | NDSI value (950 nm/1330 nm) |
|---|---|---|---|
| Coal K | Yes | 8.0 | 5.8 |
| Coal K | No | 23.1 | 25.4 |
| Coal L | Yes | 7.0 | 4.6 |
| Coal L | Yes | 6.1 | 3.5 |
| Coal L | No | 19.6 | 21.1 |

Reference Signs List

1 estimation system

2 estimation device

21 moisture model information acquisition unit

22 moisture index information acquisition unit

23 estimation unit

24 optical property information acquisition unit

25 moisture index information calculation unit

26 communication unit

27 storage unit

28 control unit

29 communication bus

3 output device

4 optical property information measurement device

S material

**Claims**

1. An estimation device configured to estimate the amount of moisture in a material as a steelmaking raw material and/or fuel for power generation,

   the estimation device comprising a moisture model information acquisition unit, a moisture index information acquisition unit, and a moisture amount estimation unit, wherein
   the moisture model information acquisition unit acquires moisture model information indicating the relation between the amount of moisture in the material and a moisture index calculated as a function of the difference between two optical properties of the material for light of two different wavelengths in a wavelength range of 800 nm to 2400 nm,
   the moisture index information acquisition unit acquires moisture index information indicating the moisture index of the material as an estimation target, and

the moisture amount estimation unit estimates the moisture amount of the material as an estimation target based on the moisture model information and the moisture index information.

2. The estimation device according to claim 1, wherein the moisture model information indicates a linear relation between the moisture index and the moisture amount of the material.

3. The estimation device according to claim 1 or 2, wherein the optical properties are absorptance, reflectance, or transmittance.

4. The estimation device according to any one of claims 1 to 3, wherein the optical properties of the material as an estimation target are measured by using a hyperspectral camera or a multispectral camera.

5. The estimation device according to claim 4, wherein

the two optical properties of the material as an estimation target are associated with two-dimensional position information,
the moisture index information acquisition unit acquires, in association with the two-dimensional position information, moisture index information indicating the moisture index of the material as an estimation target, and
the moisture amount estimation unit estimates, for each two-dimensional position, the moisture amount of the material as an estimation target based on the moisture model information and the moisture index information.

6. The estimation device according to claim 4 or 5, wherein the optical properties of the material as an estimation target are measured by the hyperspectral camera or the multispectral camera attached to an aerial vehicle.

7. An estimation device configured to estimate the content of a chemical agent in a material as a steelmaking raw material and/or fuel for power generation,

the estimation device comprising a chemical agent inclusion model information acquisition unit, an inclusion index information acquisition unit, and a content estimation unit, wherein
the chemical agent inclusion model information acquisition unit acquires chemical agent inclusion model information indicating the relation between the content or existence of inclusion of a chemical agent in the material and an inclusion index calculated as a function of the difference between two optical properties of the material for light of two different wavelengths in a wavelength range of 800 nm to 2400 nm,
the inclusion index information acquisition unit acquires inclusion index information indicating the inclusion index of the material as an estimation target, and
the content estimation unit estimates the content or existence of inclusion of a chemical agent in the material as an estimation target based on the chemical agent inclusion model information and the inclusion index information.

8. An estimation system configured to estimate the amount of moisture in a material as a steelmaking raw material and/or fuel for power generation,

the estimation system comprising a moisture model information acquisition unit, a moisture index information acquisition unit, and a moisture amount estimation unit, wherein
the moisture model information acquisition unit acquires moisture model information indicating the relation between the amount of moisture in the material and a moisture index calculated as a function of the difference between two optical properties of the material for light of two different wavelengths in a wavelength range of 800 nm to 2400 nm,
the moisture index information acquisition unit acquires moisture index information indicating the moisture index of the material as an estimation target, and
the moisture amount estimation unit estimates the moisture amount of the material as an estimation target based on the moisture model information and the moisture index information.

9. An estimation system configured to estimate the content of a chemical agent in a material as a steelmaking raw material and/or fuel for power generation,

the estimation system comprising a chemical agent inclusion model information acquisition unit, an inclusion index information acquisition unit, and a content estimation unit, wherein

the chemical agent inclusion model information acquisition unit acquires chemical agent inclusion model information indicating the relation between the content or existence of inclusion of a chemical agent in the material and an inclusion index calculated as a function of the difference between two optical properties of the material for light of two different wavelengths in a wavelength range of 800 nm to 2400 nm,
the inclusion index information acquisition unit acquires inclusion index information indicating the inclusion index of the material as an estimation target, and
the content estimation unit estimates the content or existence of inclusion of a chemical agent in the material as an estimation target based on the chemical agent inclusion model information and the inclusion index information.

10. An estimation method of estimating the amount of moisture in a material as a steelmaking raw material and/or fuel for power generation,

the estimation method comprising a moisture model information acquiring step, a moisture index information acquiring step, and a moisture amount estimating step, wherein
the moisture model information acquiring step acquires moisture model information indicating the relation between the amount of moisture in the material and a moisture index calculated as a function of the difference between two optical properties of the material for light of two different wavelengths in a wavelength range of 800 nm to 2400 nm,
the moisture index information acquiring step acquires moisture index information indicating the moisture index of the material as an estimation target, and
the moisture amount estimating step estimates the moisture amount of the material as an estimation target based on the moisture model information and the moisture index information.

11. The estimation method according to claim 10, further comprising an optical property measuring step and an optical property acquiring step, wherein

the optical property measuring step measures the two optical properties of the material as an estimation target by using a hyperspectral camera or a multispectral camera, and
the optical property acquiring step acquires the two optical properties of the material as an estimation target.

12. The estimation method according to claim 11, wherein

the optical properties are associated with two-dimensional position information,
the moisture index information acquiring step acquires, in association with two-dimensional position information, moisture index information indicating the moisture index of the material as an estimation target, and
the moisture amount estimating step estimates, for each two-dimensional position, the moisture amount of the material as an estimation target based on the moisture model information and the moisture index information.

13. The estimation method according to claim 11 or 12, wherein the optical property measuring step measures the two optical properties of the material as an estimation target by using the hyperspectral camera or the multispectral camera attached to an aerial vehicle.

14. An estimation method of estimating the content of a chemical agent in a material as a steelmaking raw material and/or fuel for power generation,

the estimation method comprising a chemical agent inclusion model information acquiring step, an inclusion index information acquiring step, and a content estimating step,
the chemical agent inclusion model information acquiring step acquires chemical agent inclusion model information indicating the relation between the content or existence of inclusion of a chemical agent in the material and an inclusion index calculated as a function of the difference between two optical properties of the material for light of two different wavelengths in a wavelength range of 800 nm to 2400 nm,
the inclusion index information acquiring step acquires inclusion index information indicating the inclusion index of the material as an estimation target, and
the content estimating step estimates the content or existence of inclusion of a chemical agent in the material as an estimation target based on the chemical agent inclusion model information and the inclusion index information.

15. An estimation program configured to estimate the amount of moisture in a material as a steelmaking raw material and/or fuel for power generation,

the estimation program causing a computer to function as a moisture model information acquisition unit, a moisture index information acquisition unit, and a moisture amount estimation unit, wherein
the moisture model information acquisition unit acquires moisture model information indicating the relation between the amount of moisture in the material and a moisture index calculated as a function of the difference between two optical properties of the material for light of two different wavelengths in a wavelength range of 800 nm to 2400 nm,
the moisture index information acquisition unit acquires optical property information indicating the moisture index of the material as an estimation target, and
the moisture amount estimation unit estimates the moisture amount of the material as an estimation target based on the moisture model information and the moisture index information.

16. An estimation program configured to estimate the content of a chemical agent in a material as a steelmaking raw material and/or fuel for power generation,

the estimation program causing a computer to function as a chemical agent inclusion model information acquisition unit, an inclusion index information acquisition unit, and a content estimation unit, wherein
the chemical agent inclusion model information acquisition unit acquires chemical agent inclusion information indicating the relation between the content or existence of inclusion of a chemical agent in the material and an inclusion index calculated as a function of the difference between two optical properties of the material for light of two different wavelengths in a wavelength range of 800 nm to 2400 nm,
the inclusion index information acquisition unit acquires inclusion index information indicating the inclusion index of the material as an estimation target, and
the content estimation unit estimates the content or existence of inclusion of a chemical agent in the material as an estimation target based on the chemical agent inclusion model information and the inclusion index information.

# Fig. 1

# Fig. 2

```
                                        2

        ┌─────────────────────────────────────┐
        │  ┌───────────────────────────────┐  │      21
        │  │ MOISTURE MODEL INFORMATION    │  │
        │  │      ACQUISITION UNIT         │  │
        │  └───────────────────────────────┘  │
        │                                     │      22
        │  ┌───────────────────────────────┐  │
        │  │  MOISTURE INDEX INFORMATION   │  │
        │  │      ACQUISITION UNIT         │  │
        │  └───────────────────────────────┘  │
        │                                     │      23
        │  ┌───────────────────────────────┐  │
        │  │       ESTIMATION UNIT         │  │
        │  └───────────────────────────────┘  │
        │                                     │      24
        │  ┌───────────────────────────────┐  │
        │  │ OPTICAL PROPERTY INFORMATION  │  │
        │  │      ACQUISITION UNIT         │  │
        │  └───────────────────────────────┘  │
        │                                     │      25
        │  ┌───────────────────────────────┐  │
        │  │  MOISTURE INDEX INFORMATION   │  │
        │  │      CALCULATION UNIT         │  │
        │  └───────────────────────────────┘  │
        └─────────────────────────────────────┘
```

# Fig. 3

# Fig. 4

ACQUIRE MOISTURE
MODEL INFORMATION  — S1

ACQUIRE MOISTURE
INDEX INFORMATION  — S2

ESTIMATE MOISTURE
AMOUNT  — S3

Fig. 5

# Fig. 6

# Fig. 7

# Fig. 8

# Fig. 9

# Fig. 10

# Fig. 11

## INTERNATIONAL SEARCH REPORT

| International application No. |
| --- |
| **PCT/JP2022/014053** |

**A.    CLASSIFICATION OF SUBJECT MATTER**

*G01N 21/27*(2006.01)i; *G01N 21/3554*(2014.01)i
FI:    G01N21/3554; G01N21/27 A; G01N21/27 F

According to International Patent Classification (IPC) or to both national classification and IPC

**B.    FIELDS SEARCHED**

Minimum documentation searched (classification system followed by classification symbols)

G01N21/00-G01N21/958

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
| --- | --- | --- |
| Y | JP 2021-047064 A (HAZAMA ANDO CORP) 25 March 2021 (2021-03-25)<br>paragraphs [0011], [0018], [0021]-[0023], [0028]-[0029], [0038]-[0040], fig. 1 | 1-16 |
| Y | JP 2001-116689 A (JAPAN SCIENCE & TECHNOLOGY CORP) 27 April 2001 (2001-04-27)<br>paragraph [0030] | 1-16 |
| Y | JP 2010-107223 A (NIPPON STEEL CORP) 13 May 2010 (2010-05-13)<br>paragraphs [0002], [0008]-[0011] | 1-16 |
| Y | JP 2016-141828 A (JFE STEEL CORP) 08 August 2016 (2016-08-08)<br>paragraphs [0009], [0012] | 1-16 |
| Y | JP 2000-146834 A (HITACHI LTD) 26 May 2000 (2000-05-26)<br>paragraphs [0008]-[0015] | 1-16 |

☐ Further documents are listed in the continuation of Box C.          ☑ See patent family annex.

| \* | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **01 June 2022** | **14 June 2022** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2022/014053**

| Patent document cited in search report | | | Publication date (day/month/year) | Patent family member(s) | Publication date (day/month/year) |
|---|---|---|---|---|---|
| JP | 2021-047064 | A | 25 March 2021 | (Family: none) | |
| JP | 2001-116689 | A | 27 April 2001 | (Family: none) | |
| JP | 2010-107223 | A | 13 May 2010 | (Family: none) | |
| JP | 2016-141828 | A | 08 August 2016 | (Family: none) | |
| JP | 2000-146834 | A | 26 May 2000 | US 6633035 B1<br>column 2, line 5 to column 4, line 34<br>WO 2000/028303 A1<br>EP 1128178 A1<br>KR 10-2001-0083943 A | |

Form PCT/ISA/210 (patent family annex) (January 2015)

**REFERENCES CITED IN THE DESCRIPTION**

**Patent documents cited in the description**

- JP 2008050076 A